(19) **Europäisches Patentamt European Patent Office Office européen des brevets**

(11) **EP 2 049 587 B1**

(12) **EUROPEAN PATENT SPECIFICATION**

(45) Date of publication and mention
of the grant of the patent:
**06.03.2013 Bulletin 2013/10**

(21) Application number: **07776860.4**

(22) Date of filing: **07.05.2007**

(51) Int Cl.:
*C08K 7/16* (2006.01)       *C11D 3/37* (2006.01)
*C11D 3/50* (2006.01)       *C11D 17/04* (2006.01)
*C11D 17/00* (2006.01)      *A61K 8/11* (2006.01)
*A61Q 13/00* (2006.01)      *A61K 8/02* (2006.01)

(86) International application number:
**PCT/US2007/011064**

(87) International publication number:
**WO 2007/130685 (15.11.2007 Gazette 2007/46)**

(54) **FILMS WITH MICROCAPSULES**

FOLIEN MIT MIKROKAPSELN

FILMS PRÉSENTANT DES MICROCAPSULES

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **05.05.2006 US 798158 P**

(43) Date of publication of application:
**22.04.2009 Bulletin 2009/17**

(73) Proprietor: **The Procter & Gamble Company Cincinnati, OH 45202 (US)**

(72) Inventors:
• **DENOME, Frank, William**
  **Cincinnati, Ohio 45211 (US)**
• **BERGES CABRERA, Tania, Edmee**
  **Cincinnati, Ohio 45209 (US)**
• **WAHL, Errol, Hoffman**
  **Cincinnati, Ohio 45242 (US)**

• **BROWN, Jodi, Lee**
  **Cincinnati, Ohio 45208 (US)**
• **FOSSUM, Renae, Dianna**
  **Middletown, Ohio 45044 (US)**
• **CATALFAMO, Vincenzo**
  **Cincinnati, oh 45236 (US)**
• **GIZAW, Yonas**
  **Cincinnati, Ohio 45246 (US)**
• **BOEKLEY, Lois, Jean**
  **Cincinnati, OH 45241 (US)**

(74) Representative: **Mather, Peter Geoffrey NV Procter & Gamble Services Company SA 100 Temselaan 1853 Strombeek-Bever (BE)**

(56) References cited:
**WO-A-94/02377       WO-A-2004/020566**
**DE-A1- 3 447 833    US-A- 5 254 598**

## Description

BACKGROUND OF THE INVENTION

The home laundering process provides consumers with the opportunity to treat fabrics with a multitude of materials which can impart desirable benefits to the fabric during the wash and/or rinse cycle. Scent experience is one of these desired benefits. One problem with conventional laundering products is that the scent experience is limited by the types of scents available in laundry detergents and/or fabric conditioners. Another problem with conventional laundering products is that the intensity of the scent experience is often determined by the amount of detergent and/or fabric softener used. Yet another problem with conventional laundering products is the longevity of the scent experience on fabrics; i.e., the scent does not last long enough.

Conventional laundering products provide consumers with limited scent options. One problem is that conventional laundering products such as detergents and fabric softeners are often sold with different types of scents which may be incompatible when used in combination. Recent advancements allow consumers to select different laundry products with coordinated scents, for example, detergent and fabric softener having the same scent or similar scents. Despite the many different types of scented laundering products available, there remains a need to provide consumers with an easy and affordable way to further customize their scent experiences without being limited to scents or perfumes available in detergents and/or fabric softeners.

Conventional laundering products provide limited ability for consumers to control the intensity of the scent deposited on their fabrics. Overdosing and underdosing are methods used by consumers to control the intensity of the scent deposited on their fabrics. One problem with controlling scent intensity by overdosing and underdosing is that other benefits such as cleansing and softening may require specific dosage amounts to be effective. In one case, significant overdosing of a conventional fabric softener can reduce the water absorbency of cotton terry towels. As such, there remains a need for new ways to allow consumers to control the intensity of their scent experience.

A number of compositions and articles exist for delivering desirable benefits to the fabric during laundering. *See e.g.* U.S. Patent No. 3,936,538 to Marshall et al.; U.S. Patent No.

**[0005]** 7,049,274 to Renade et al.; U.S. Patent No. 7,015,186 to Aussant et al.; U.S. Patent Application No. 2005/0226826 to Eason et al.; and U.S. Patent Application Publication No. 2006/0019866 to Briggs et al. Despite these and other improvements, there remains a need to provide consumers with a fabric treatment composition which will allow them to customize and control their scent experience through the wash and/or rinse. Further, there still exits a need to provide longer lasting scent on fabrics.

SUMMARY OF THE INVENTION

In one aspect of the present invention, there is provided an article as defined in claim 1.

DETAILLED DESCRIPTION OF THE INVENTION

**Functionalized substrate**

Functionalized Substrate

According to one aspect of the invention, there is provided a functionalized substrate comprising a composition susceptible to aqueous attack and a plurality of microcapsules encapsulating a functional composition, wherein the plurality of microcapsules is incorporated with the functionalized substrate. As used herein, incorporated with the functionalized substrate means that the plurality of microcapsules are either within the functionalized substrate, touching the surface of the functionalized substrate, at least partially on the surface of the functionalized substrate, or otherwise connected to the functionalized substrate. In another embodiment, the plurality of microcapsules is dispersed throughout the Functionalized substrate. In yet another embodiment, the plurality of microcapsules is uniformly dispersed throughout the functionalized substrate.

The functionalized substrates of the present invention have a multitude of applications and methods of use. One application for functional substrates described herein is in the field of fabric care. One method of use provides for administering the functionalized substrate into the basin of an automatic or manual laundry washing machine, either in the wash cycle and/or the rinse cycle. Another method of use provides for administering the functionalized substrate in the dryer. Yet another method of use is administering the functionalized substrates into a tub, basin, bucket or container in hand laundering situations. The functionalized substrates can be added in the hand washing step, rinsing step or both, but preferably in the rinsing step.

Functionalized substrates according to the present invention can be used to customize the user's scent experience when laundering fabrics. In one method of customizing the scent experience, the user chooses one or more functionalized substrates that comprise a perfume that has a similar or complimentary scent as the laundry detergent and/or fabric softener that is used. In yet another method, one or more functionalized substrates are used in combination with unscented laundry detergent and/or fabric softener. In yet another method, one or more functionalized substrates are used without any added laundry detergent or fabric softener.

Additionally, functionalized substrates according to the present invention can be used to control the intensity of scent when laundering fabrics. A user may administer one or more substrates with scented or unscented laundry products to thereby control the intensity of scent on their fabrics. In one embodiment, the article further comprises instructions about these and other methods of use. In yet another embodiment, functionalized substrates according to the present invention can be used to provide other benefits to fabrics in addition to or instead of scent. In yet another embodiment, the user may administer a plurality of functionalized substrates where each functionalized substrate has different perfumes to create their own customized scent experience. Further, using the functionalized substrates of the present invention can allow users to increase the longevity of scent on their laundered fabrics.

**Functionalized Substrate Composition**

**1. Aqueous Attack**

The functional substrate according to the present invention comprises a composition which is susceptible to aqueous attack. As used herein, aqueous attack means that the composition when contacted with an aqueous solution dissolves, deforms, disintegrates, solubilizes, or otherwise undergoes physical degradation. The term "aqueous solution" should be broadly interpreted for the purpose of this invention, including any mixture comprising water. In one embodiment, the water content of the aqueous solution is at least about 10%, alternatively at least 20%, alternatively at least about 30%, alternatively at least about 40%, alternatively even greater than about 99% by weight of the aqueous solution. In another embodiment, the aqueous solution comprises one or more functional materials. In yet another embodiment, the aqueous solution comprises slurries and dispersions (liquid/solid), foams (liquid-gas), gels, and emulsions (liquid/liquid) and mixtures thereof.

**2. Compositions Susceptible to Aqueous Attack**

In one embodiment, the composition susceptible to aqueous attack comprises water-soluble materials, partially water-soluble materials, water-dispersible materials, water-disintegrating materials, and mixtures thereof.

a. Water-Soluble and Partially Water-Soluble Materials

In one embodiment of the present invention, the composition susceptible to aqueous attack comprises a water-soluble or partially water-soluble material. As used herein, water-soluble materials include partially water-soluble materials. Where a water-soluble material is used, the functionalized substrate has a water-solubility of at least 50%, alternatively at least 75%, or even at least 95%, as measured by the Water-Solubility Method as provided herein.

Water-Solubility Method: 50 grams $\pm$ 0.1 gram of functionalized substrate material is added in a pre-weighed 400 ml beaker and 245ml $\pm$ 1ml of distilled water (temperature = 15·56°C (60°F) is added. This composition is stirred vigorously on a magnetic stirrer set at 600 rpm, for 30 minutes. Then, the mixture is filtered through a folded qualitative sintered-glass filter with a pore size as defined above (max. 20 micron). The water is dried off from the collected filtrate by any conventional method, and the weight of the remaining material is determined (which is the dissolved or dispersed fraction). The % water-solubility is then calculated.

Suitable water-soluble materials include water-soluble polymeric materials (polymers) which can be formed into a film or sheet or laminate or extruded (or extruded or pressed into a 3-deminsional shape) or blown into a foam. In one embodiment, the level of polymer in the functionalized substrate is at least about 5% by weight of said functionalize substrate. In another embodiment, the level of polymer is from about 10% to about 99%, alternatively from about 15% to about 90%, alternatively from about 20 % to about 85%, by weight of said functionalized substrate. Films formed of polymers can, for example, be obtained by casting, blow-moulding, extrusion or blown extrusion of the polymeric material, as known in the art. Non-limiting examples of suitable foams formed of polymers and methods of forming such foams have been disclosed in U.S. Patent No. 7,056,877.

Examples of polymers, copolymers or derivatives thereof suitable for use as water-soluble material include but are not limited to polyvinyl alcohols (PVA), modified PVAs; polyvinyl pyrrolidone; PVA copolymers such as PVA/polyvinyl pyrrolidone; partially hydrolyzed polyvinyl acetate; polyalkylene oxides such as polyethylene oxide; acrylamide; acrylic acid; cellulose, alkyl cellulosics such as methyl cellulose, ethyl cellulose and propyl cellulose; cellulose ethers; cellulose esters; cellulose amides; polyvinyl acetates; polycarboxylic acids and salts; polyaminoacids or peptides; polyamides;

polyacrylamide; copolymers of maleic/acrylic acids; polysaccharides including starch, modified starch; gelatin; alginates; xyloglucans, other hemicellulosic polysaccharides including xylan, glucuronoxylan, arabinoxylan, mannan, glucomannan and galactoglucomannan; and natural gums such as pectin, xanthan, and carrageenan, locus bean, arabic, tragacanth; and combinations thereof. In one embodiment the polymer comprises polyacrylates, especially sulfonated polyacrylates and water-soluble acrylate copolymers; and alkylhydroxy cellulosics such as methylcellulose, carboxymethylcellulose sodium, modified carboxy-methylcellulose, dextrin, ethylcellulose, propylcellulose, hydroxyethyl cellulose, hydroxypropyl methylcellulose, maltodextrin, polymethacrylates. In yet another embodiment the polymer comprises PVA; PVA copolymers; hydroxypropyl methyl cellulose (HPMC); and mixtures thereof.

Where the composition comprises PVA, the functionalized substrate comprises a rapid dissolution rate, as defined herein, where the functionalized substrate at least partially dissolves in an aqueous solution at cold temperatures, i.e., less than about 4·44°C (40°F) or 10°C (50°F) during the wash cycle and/or the rinse cycle. Typical wash and/or rinse cycles should take about 10 minutes, alternatively about 5 minutes. In one embodiment, the entire functionalized substrate dissolves in during the wash and/or rinse cycles. In one embodiment, PVA is mixed or blended with another polymer to obtain the desired dissolution rate. It is believed that selecting polymers based on average molecular weight and/or degree of hydrolysis allows for different dissolution rates.

Without intending to be bound by theory, it is believed that selecting PVAs based on average molecular weights allows for increased dissolution rates in cold temperatures. In one embodiment, the PVA comprises an average molecular weight (i.e., mean weights of the molar masses) from about 6,000 to about 78,000, alternatively greater than about 78,000.

Additionally, it has been believed that selecting PVAs based on degree of hydrolysis allows for increased dissolution rates in cold temperatures. In another embodiment, the PVA comprises varying degrees of hydrolysis. In one embodiment, the PVA comprises less than about 90%, alternatively less than about 85%, and alternatively less than about 80% hydrolyzed polymer, but will be more than about 60% and alternatively at least about 70% hydrolyzed polymer. In another embodiment, the PVA is from about 60 mol% to about 98 mol% hydrolyzed, alternatively about 80 mol% to about 90 mol% hydrolyzed.

In one embodiment, the composition comprises a combination or mixture of more than one of PVA resins. In another embodiment, the PVA resin comprises a weight average molecular weight range of about 55,000 to about 65,000 and a number average molecular weight range of about 27,000 to about 33,000. Additional suitable resins include poly (ethylene oxide) and cellulose derived water-soluble carbohydrates, the former being produced by Union Carbide, Inc. and sold under the tradename Polyox; the latter being produced by Dow Chemical, Inc. and sold under the Methocel trademark. Typically, cellulose derived water-soluble polymers are not readily melt processable. In yet another embodiment, the composition comprises melt processable PVA resins. Suitable melt processable PVA resins are produced by Texas Polymer Services, Inc., tradenamed Vinex, under license from Air Products and Chemicals, Inc. and by MonoSol LLC of Indiana, U.S., among others.

In yet another embodiment, the composition comprises polymers having low molecular weight and/or chemically modified polylactides; such polymers have been produced by Chronopol. Inc. and sold under the Heplon trademark.

In one embodiment, the functionalized substrate comprises a PVA film. Suitable PVA films are known under the trade reference MonoSol M8630, as sold by MonoSol. Other films suitable for use herein include films known under the trade reference PT film or the K-series of films supplied by Aicello, or VF-HP film supplied by Kuraray.

b. <u>Mixtures/Blends of Polymers</u>

In one embodiment of the present invention, the composition comprises a mixtures (or blend) of polymers. As defined herein, mixtures and blends can be used interchangeably to represent a combination of two or more compositions to form a third composition, the mixture or blend. Without intending to be bound by theory, it is believed that blends of polymers are beneficial in controlling the mechanical and/or dissolution properties of the functionalized substrate, depending on the application thereof and the required needs. In one embodiment, a blend of polymers is used, having different weight average molecular weights. For example, a blend of 1) PVA or a copolymer thereof, having a weight average molecular weight of about 10,000 to about 40,000, alternatively about 20,000, and 2) PVA or copolymer thereof, having a weight average molecular weight of about 100,000 to about 300,000, alternatively about 150,000.

In another embodiment, the composition comprises a polymer blend compositions, for example comprising hydrolytically degradable and water-soluble polymer blend such as polylactide and PVA. Hydrolytically degradable and water soluble polymer blends can be obtained by mixing polylactide and PVA, typically comprising from about 1% to about 35% by weight polylactide and from about 65% to about 99% by weight PVA. These types of polymer blends can water-soluble and/or water-dispersible.

It is further believed that specific blends of polymers provide dissolving films or foams with dissolution rates of the present invention, which can be produced with good mechanical properties for subsequent handling and converting into manufactured articles. For instance, a blend containing at least two types of polymers that have disparate molecular weights, can be used to prepare substrate that dissolves at a dissolution rate as disclosed herein under cold water conditions. In one embodiment, such blends contain at least a first polymer comprising a molecular weight greater than about 50,000, alternatively greater than about 60,000, and alternatively greater than about 70,000, and a second polymer or mixture of polymers comprising an average molecular weight of less than about 30,000, alternatively less than about 15,000, and alternatively less than about 10,000.

c. PVA Blends

In one embodiment, the composition comprises a polymer blend. In another embodiment the polymer blend comprise two or more PVAs; PVA and polyvinyl pyrrolidone; PVA and polyethylene oxide; PVA and hydroxymethyl cellulose; PVA and hydroxyethyl cellulose; polyvinyl; and PVA and hydroxypropyl methylcellulose. Additional suitable polymer blends include pyrrolidone and hydroxyethyl cellulose; polyvinyl pyrrolidone and polyethylene oxide; polyethylene oxide and hydroxyethyl cellulose. In yet another embodiment, the blends ratio is 80/20, 60/40 and 50/50 by weight.

Blends of high and low molecular weight polymers at ratios of 80/20, 60/40, and 50/50 mixtures of low to high molecular weight polymers can be evaluated for specific applications. In one embodiment, the composition comprises a blend of at least one PVA having a molecular weight of about 78,000 and higher and a second PVA about 6,000 or lower. This embodiment has been found to produce a film which dissolves at a rate as defined herein under cold water conditions. Further, a low percentage of the higher molecular weight PVA, i.e. less than about 50% alternatively less than about 40%, and alternatively less than about 30%, will produces a film with adequate strength for converting into pouches, sachets or coatings. A high percentage of higher molecular weight PVA, i.e. greater than about 50%, alternatively greater than about 60% and alternatively greater than about 70%, will provide the improved strength and elasticity that is desired for vacuum forming operations, but such higher percentages of high molecular weight polymers are typically accompanied by increasingly higher dissolution times.

One embodiment of the present invention provides a substrate comprising a specific PVA blend which dissolves at a rate as defined herein. This PVA blend comprises from about 60% to about 95% of PVA of an average molecular weight from about 3,000 to about 30,000 and from about 5% to about 40% of PVA of an average molecular weight from about 30,000 to about 200,000. The degree of hydrolysis in this PVA blend can be less than about 90 mol%, alternatively less than about 85 mol%, and alternatively less than about 80 mol%. It has been found that a substrate sample, weighing from about 2 grams to about 1 gram, comprising this blend of PVAs can dissolve in a beaker of water at a temperature below about 68° F in less than about 5 minutes with agitation.

d. Water-dispersible materials and water-disintegrating materials.

In another embodiment, the composition susceptible to aqueous attack comprises a water-dispersible material. Non-limiting examples of such water-dispersible materials include those disclosed in U.S. Patent Publication No. 2006/0293419 A1, published December 28, 2006, U.S. Patent No. 7,094,817, published April 22, 2006, WO 0131103 A3, published May 3, 2001, U.S. Patent No. 6,211,309, published April 3, 2001, and U.S. Patent No. 5,224,601, published July 6, 1993.

In yet another embodiment, the composition susceptible to aqueous attack comprises a water-disintegrating material. Non-limiting examples of such water disintegrating materials include those disclosed in Japanese Patent No. 3525174 assigned to Chisso Corp. of Japan (Japanese Patent Application No. H09-279457) and Japanese Patent Application No. H10-008364, assigned to Chisso Corp of Japan.

**A Plurality of Microcapsule**

The functionalized substrate of the present invention further comprises a plurality of microcapsules. The term "microcapsule" is used herein the broadest sense and includes the encapsulation of perfume or other materials or actives in small capsules (i.e., microcapsules), typically having a diameter less than about 300 microns. Typically, these microcapsules comprise a spherical hollow shell of water insoluble or at least partially water insoluble material, typically polymer material, within which the active material, such as perfume, is contained. Non-limiting examples of microcapsules are available in the following references: US 2003/215417 A1; US 2003/216488 A1; US 2003/158344 A1; US 2003/165692 A1; US 2004/071742 A1; US 2004/071746 A1; US 2004/072719 A1; US 2004/072720 A1; EP 1,393,706 A1; US 2003/203829 A1; US 2003/195133 A1; US 2004/087477 A1; US 2004/0106536 A1; US 6,645,479; US 6,200,949; US 4,882,220; US 4,917,920; US 4,514,461; US RE 32,713; US 4,234,627.

The plurality of microcapsules comprises a friable microcapsule. Friability refers to the propensity of the microcapsules to rupture or break open when subjected to direct external pressures or shear forces. For purposes of the present invention, a microcapsule is "friable" if, while attached to fabrics treated therewith, the microcapsule can be ruptured by the forces encountered when the capsule-containing fabrics are manipulated by being worn or handled (thereby releasing the contents of the capsule). The plurality of microcapsules may comprise a moisture-activated microcapsule such as beta-cyclodextrin. The plurality of microcapsules may comprise a heat-activated microcapsule. As defined herein, a heat-activated microcapsule is one that ruptures by body heat and/or by the heat in a machine dryer. Non-limiting examples of additional microcapsules include wax comprising microcapsule such as those described in U.S. Pat. No. 5,246,603 and starch-based microcapsule also described in U.S. Pat. No. 5,246,603.

Microcapsules may be prepared using a range of conventional methods known to those skilled in the art for making shell capsules, such as interfacial polymerization, and polycondensation. See e.g., US 3,516,941, US 4,520,142, US 4,528,226, US 4,681,806, US 4,145,184; GB 2,073,132; WO 99/17871; and MICROENCAPSULATION: Methods and Industrial Applications Edited by Benita and Simon (Marcel Dekker, Inc. 1996). It is recognized, however, that many variations with regard to materials and process steps are possible. Non-limiting examples of materials suitable for making shell of the microcapsule include urea-formaldehyde, melamine-formaldehyde, phenol-formaldehyde, gelatin, gelatin/gum arabic blend, polyurethane, polyamides, or combinations thereof.

In one embodiment, the shell of the microcapsules comprises an aminoplast resin. A method for forming such shell capsules includes polycondensation. Aminoplast resins are the reaction products of one or more amines with one or more aldehydes, typically formaldehyde. Non-limiting examples of suitable amines include urea, thiourea, melamine and its derivates, benzoguanamine and acetoguanamine and combinations of amines. Suitable cross-linking agents (e.g., toluene diisocyanate, divinyl benzene, butanediol diacrylate etc.) may also be used and secondary wall polymers may also be used as appropriate, e.g., anhydrides and their derivatives, particularly polymers and co-polymers of maleic anhydride as disclosed in US 2004/0087477 A1. In another embodiment, the microcapsule shell is formed by cross-linking aldehydes and amine functionalities.

In one embodiment, the plurality of microcapsules comprises a mean diameter in the range from about 1 micrometer to about 100 micrometers, alternatively from about 5 micrometers to about 80 micrometers, alternatively from about 10 micrometers to about 75 micrometers, and alternatively between about 15 micrometers to about 50 micrometers. The particle size distribution can be narrow, broad or multimodal.

In another embodiment, the plurality of microcapsules vary in size having a maximum diameter between about 5 microns and about 300 microns, alternatively between about 10 microns and about 200 microns. Without intending to be bound by theory, it is believed that as the microcapsule diameters approach about 300 microns, (e.g. about 250 microns), a reduction in the number of microcapsules entrained in the fabric may be observed.

In another embodiment, the plurality of microcapsules comprises an average shell thickness from about 0.1 micron to about 50 microns, alternatively from about 1 micron to about 10 microns.

## 1. Functional Composition

The functional substrate of the present invention comprises a plurality of microcapsules encapsulating a functional composition. As used herein, functional composition means a composition which comprises one or more functional materials. As used herein functional material means any material that performs a function or delivers a benefit after dissolution of the substrate, or which modify the physical or chemical properties of the substrate, other than aesthetic appearance. For example inks, decorative dyes and pigments are not considered functional materials. However, a hueing dye for improved whiteness appearance of fabrics is considered a functional material.

The functionalized substrate of the present invention is suitable for loading high levels functional compositions. In one embodiment, the functionalized substrate comprises at least about 1 weight percent, alternatively at least about 5 weight percent, alternatively at least about 10 weight percent, alternatively at least about 25 weight percent, and alternatively at least about 50 weight percent of a functional composition, by weight of the functionalized substrate. In yet another embodiment, a coating of a functional composition can be applied to a functional substrate already containing microcapsules. The coating can contain the same or different microcapsules. Non-limiting examples of suitable coatings are disclosed in EP 2021172 to Wahl et al.

In one embodiment, the functional composition comprises one or more functional materials, including, but not limited to: flavors, perfumes, softening agents, anti-static agents, crisping agents, water/stain repellents, stain release agents, refreshing agents, anti-microbial agents, disinfecting agents, wrinkle resistant agents, wrinkle release agents, odor resistance agents, malodor control agents, abrasion resistance and protection agents, solvents, insect/pet repellents, wetting agents. UV protection agents, skin/fabric conditioning agents, skin/fabric nurturing agents, color protection agents, dye fixatives, dye transfer inhibiting agents, silicones, preservatives and anti-microbials, fabric shrinkage-reducing agents, perfume microcapsules, brighteners, hueing dyes, bleaches, chelants, antifoams, anti-scum agents, whitening agents, and combinations thereof. Microcapsules encapsulating these and other commonly used functional materials can be used in accordance with the present invention.

In another embodiment, the functional material comprises a perfume raw material, silicone oils and silicone waxes, waxes, hydrocarbons, isoparaffins (e.g., Permethyls available from Chesham Specialty Ingredients LTD), higher fatty acids, essential oils, lipids, skin coolants, vitamins, sunscreens, antioxidants, glycerin, catalysts, bleaches and bleach particles, bleach activators, bleach catalysts, soil suspending polymers, wetting agents, silicon dioxide particles, malodor reducing agents, dyes, brighteners, antibacterial actives, antiperspirant actives, cationic polymers, polydimethylsiloxane (PDMS or derivatized PDMS; one example is silicone polyols), aminofunctional silicones, sucrose polyesters, polyglycerol esters, polyethylene waxes, vitamin E, enzymes, amino acids, shea butter, aloe vera, petrolatum, retinol, cucumber extracts, chamomile extracts, almond milk, silk protein, keratin protein and keratin amino acids, natural soap, eucalyptus, natural oat, sea minerals, lavender, rose, vanilla extract, linen flower, citrus, lemon, lime, orange, and mixtures thereof.

Other suitable functional materials include laundry cleaning actives, barrier agents, solubility modifiers, fabric softening actives including cationic surfactants, quaternary ammonium compounds, and antistatic actives, silicones, antifoams and mixtures thereof.

**2. Perfume Microcapsules**

The functional material comprises a perfume composition. Non-limiting examples of suitable perfume compositions include blooming perfumes, perfume oils, and perfume raw material comprising alcohols, ketones, aldehydes, esters, ethers, nitriles alkenes, and mixtures thereof.

In another embodiment, the plurality of microcapsules comprise a loading/complexation level of from about 50% to about 90%, alternatively from about 60% to about 85%, alternatively from about 65%% to about 75%, by weight of a perfume composition. This loading/complexation property of the present invention is advantageous versus other technologies, such as beta-cyclodextrin. It is believed that advantages may include, but are not limited to, one or more of the following: (i) the ability to use a reduced total perfume level, e.g., in neat perfume (direct add); in perfume microcapsules; or combinations thereof; (ii) avoiding cost in processing and lost material through processing; (iii) delivering a high level of perfume while not affecting process product disposition or process parameters or product stability or product physical properties (one example is viscosity); and (iv) delivering a high level of perfume to fabric while avoiding a high level of neat product odor, which can be a consumer negative; (v) delivering improved fabric odor longevity performance compared to neat perfume; and (vi) delivering improved odor from fabrics under stress conditions (one example is while wearing clothing during physical activity or exercise).

Another aspect of the invention provides a functional composition comprising a perfume composition comprising at least one of the following: (a) perfume microcapsule comprising a perfume carrier and an encapsulated perfume composition, wherein said perfume microcapsule is selected from a moisture-activated microcapsule, a heat-activated microcapsule, a friable microcapsule, or mixtures or combinations thereof.; (b) a pro-perfume; (c) a low odor detection threshold perfume ingredients; (d) neat perfume; and (e) combinations thereof. In one embodiment, the article is free or substantially free of any one or more of the aforementioned perfume components. A non-limiting example of a moisture-activated perfume microcapsule includes one that comprises cyclodextrin.

Suppliers of suitable microcapsules include Appleton of Appleton, WI, International Flavors & Fragrances (IFF) of New York, NY, Reed Pacific of Australia. Examples of suitable microcapsules include Perfume Microcapsules (PMCs) from Appleton, EVERLAST from IFF and WIZARD from Reed Pacific.

In one embodiment, the perfume composition comprises a single perfume raw material. In another embodiment, the perfume composition comprises more than one perfume raw material, selected to provide a specific scent experience. In yet another embodiment, the perfume composition comprises a blooming perfume composition. In one embodiment, the blooming perfume composition comprises from about 3 to about 300 different perfume ingredients.

Once friable microcapsules containing a perfume composition of the present invention have been attached to fabrics being treated, it is, necessary to manipulate the treated fabrics in a manner sufficient to rupture the microcapsules and thereby release the perfume composition. Microcapsules of the type utilized herein have friability characteristics such that the ordinary fabric manipulation which occurs when the treated fabrics are worn or used is sufficient for the attached microcapsules to impart a noticeable odor to the fabric. A significant number of attached microcapsules can be broken by the normal forces encountered when treated garments are worn. For fabric articles which are not worn, the normal household handling operations such as folding, crumpling etc. can serve as fabric manipulation sufficient to rupture the attached microcapsules. Even broken or ruptured microcapsules can provide a perfume "sink" wherein the fragrance is slowly released over time and gives longevity of odor on fabric benefit. The perfume composition of the present invention surprisingly maximizes the effect of the microcapsules bursting by providing a perfume composition that "blooms" upon the microcapsules rupturing.

a. Blooming Perfume

The present invention is based, in part, upon the discovery that the blooming perfume compositions of the present invention maximizes the opportunity for the consumer of a unique scent experience during the wearing, folding, and even after storage of laundry when fabric deposited with friable microcapsules are ruptured. In one embodiment, the perfume microcapsule encapsulates a blooming perfume composition, wherein the blooming perfume composition, in the absence of water, comprises from about 5% to about 95%, alternatively from about 20% to about 90%; alternatively from about 30% to about 85%, and alternatively from about 40% to about 80%, by the total weight of the perfume microcapsule and the encapsulated perfume composition, also in absence of water.

The term "blooming perfume composition" as used herein means a perfume composition that comprises at least about 25%, alternatively at least about 35%, alternatively at least about 45%, alternatively at least about 55%, alternatively at least about 65%, by weight of the perfume composition, of blooming perfume ingredients, wherein the blooming perfume ingredients are those having a boiling point (B.P.) equal to or lower than about 250°C, wherein the B.P. is measured at the normal standard pressure.

The boiling points of many perfume ingredients are given in, e.g., "Perfume and Flavor Chemicals (Aroma Chemicals)," S. Arctander, published by the author, 1969. Other boiling point values can be obtained from different chemistry handbooks and databases, such as the Beilstein Handbook, Lange's Handbook of Chemistry, and the CRC Handbook of Chemistry and Physics. When a boiling point is given only at a different pressure, usually at a pressure lower than the standard pressure (760 mm Hg), the boiling point at standard pressure can be approximately estimated by using boiling point-pressure monographs, such as those given in "The Chemist's Companion," A. J. Gordon and R. A. Ford, John Wiley & Sons Publishers, 1972, pp. 30-36. When applicable, the boiling point values can also be calculated by computer programs, based on molecular structural data, such as those described in "Computer-Assisted Prediction of Normal Boiling Points of Pyrans and Pyrroles," D. T. Stanton et al, J. Chem. Inf. Comput. Sci., 32 (1992), pp. 306-316, "Computer-Assisted Prediction of Normal Boiling Points of Furans," Tetrahydrofurans, and Thiophenes," D. T. Stanton et al, J. Chem. Inf. Comput. Sci., 31 (1992), pp. 301-310, and references cited therein, and "Predicting Physical Properties from Molecular Structure," R. Murugan et al, Chemtech, June 1994, pp. 17-23.

Non-limiting examples of blooming perfume ingredients that are useful in the articles of the present invention are given in U.S. Pat. Pub. No. 2005/0192207 A1, ¶¶ 29 - 31.

In one embodiment, the blooming perfume composition of the present invention comprises at least about 3 different blooming perfume ingredients, alternatively at least about 4 different blooming perfume ingredients, alternatively at least about 5 different blooming perfume ingredients, and alternatively at least about 6 different blooming perfume ingredients.

In another embodiment, the perfume comprises enduring perfume ingredients that have a boiling point of about 250°C or higher and a ClogP of about 3.0 or higher, more preferably at a level of at least about 25%, by weight of the perfume. Suitable perfumes, perfume ingredients, and perfume carriers are described in U.S. Patent Number 5,500,138; and U.S. Patent Application Number 2002/0035053 A1.

In the perfume art, some materials having no odor or very faint odor are used as diluents or extenders. Non-limiting examples of these materials are dipropylene glycol, diethyl phthalate, triethyl citrate, isopropyl myristate, and benzyl benzoate. These materials are used for, e.g., diluting and stabilizing some other perfume ingredients. For purposes of this invention, these materials are not counted as a "blooming perfume ingredient."

In one embodiment, substantive perfume ingredients, which can be used as part of blooming perfume compositions in articles of the present invention, are those having a B.P. higher than about 250°C. Non-limiting examples of such perfume ingredients include those described in U.S. Pat. Pub. No. 2005/0192207 A1, published Sep. 1, 2005, ¶ 36.

### 3. Other Functional Materials

a. <u>Laundry Cleaning Actives</u>

In one embodiment, the functional material comprises a laundry cleaning active. Laundry cleaning actives are substances which play an active role in the cleaning process of laundry. Cleaning actives include, but are not limited to, substances such as detersive surfactants (anionic, nonionic, cationic, zwitterionic, and amphoteric surfactants), builders (inorganic and organic builder substances), bleaches, bleach activators, bleach stabilizers, bleach catalysts, enzymes, soil suspending or dispersing polymers, chelants, or combinations thereof, without the term being restricted to these substance groups. In one embodiment, the term "cleaning active" may be free or substantially free of one or more above identified actives. In one embodiment, the laundry cleaning active is not encapsulated in the microcapsule.

Barrier agents perform a protective function. For example, they can protect mutually incompatible cleaning actives from one another, cleaning actives or solubility modifiers from the outside environment, the film from the external environment, etc. They can also modify the feeling at touch of the film and/or functional materials. They can make substrates more pleasant to the touch. Suitable barrier agents may include zeolite, bentonite, talc, mica, kaolin, silica, silicone, starch, cyclodextrin, varnish, shellac, lacquer, polyolefins, paraffins, waxes, polyacrylates, polyurethanes, PVA, polyvinyl acetate, UV absorbers ( see e.g., McCutcheon's Volume 2, Functional Materials, North American Edition, published by the Manufacturing Confectioner Publishing Company (1997)), fluorescent dyes, (see e.g., EP 1,141,207, US 5,082,578), or combinations thereof.

In one embodiment, the functional composition may comprise one or more of the following material(s): soil release polymer, anti-oxidants, colorants, preservatives, optical brighteners, opacifiers, stabilizers such as guar gum and polyethylene glycol, anti-shrinkage agents, anti-wrinkle agents, soil release agents, fabric crisping agents, reductive agents, spotting agents, germicides, fungicides, anti-corrosion agents, antifoam agents, hueing dyes, and the like. In one embodiment, the functional composition is free or substantially free of any one or more of the above-identified optional components.

In another embodiment the functionalized substrate comprises an aesthetic agent. The aesthetic agent can have ornamental purposes and can denote the presence of functional materials on the film. It can also signal when a functional material is released or a product "end of life" via a change in color and/or appearance/disappearance of graphics, patterns, trademarks, etc.

b. Fabric Softening Actives

In one embodiment of the invention, the functional material comprises a fabric softening active. Such fabric softening actives are preferably those effective in a "wash-added" (verses a rinse-added) context, although the use of quaternary ammonium compounds are not excluded as functional materials from this invention. Non-limiting examples include silicone, fatty acid, fatty esters, polyglycerol esters, polyethylene waxes, sucrose esters, clays, triglycerides, cationic starches, and cationic polymers. Coacervates with silicone and other softening actives in these co-pending patent applications are included by reference: U.S. 2005/0020476 A1 and U.S. 2006/0217288 A1. In one embodiment, the fabric softening active is not encapsulated by the microcapsule. In another embodiment, the functionalized substrate is free or substantially free of a fabric softening active. Additional suitable fabric softening actives are disclosed in U.S. 2006/0058214 A1.

c. Deposition Agents

In one embodiment, the functional material comprises a deposition agent including, but not limited to I) non-quaternary materials that are (a) acyclic polymers or copolymers having nitrogen moieties in the backbone or in the pendant groups, or (b) vinyl polymers or copolymers having nitrogen heterocyclics in the pendant groups: II) non-polysaccharide polyquatemiums and other polymeric cationic quaternary materials; and mixtures thereof.

The deposition agents suitable for use herein are polymeric materials having a weight average molecular weight generally in the range from about 1000 to about 1,000,000, or from about 1000 to about 200,000, or from about 2500 to about 1,000,000, or from about 5000 to about 500,000. In one embodiment, the deposition aid is polyacrylamide or derivatives thereof, the weight average molecular weight from about 1,000,000 to about 15,000,000.

When present, each deposition agent comprises, based on total composition weight, from about 0.01 % to about 20%, alternatively from about 0.1-% to about 15%, alternatively from about 0.2% to about 10 wt %, and alternatively from about 0.5% to about 5%.

Examples of suitable deposition agents are acyclic polymers or copolymers derived from monomers having nitrogen moieties, including but not limited to, amine, imine, amide, imide, acrylamide, methacrylamide, amino acid, and mixtures thereof. Additional suitable deposition agents are disclosed in U.S. Patent Publication No. 2006/0058214 A1, and WO 2008/120176.

d. Cationic Polymers

The term "cationic polymer" is used herein in its broadest sense to include any polymer (including in one embodiment, a cationic surfactant) which has a cationic charge. Some cationic polymers can function as deposition aids as described in the next section; or alternatively, provide fabric care benefits on their own such as antiabrasion effects to improve the appearance of colored fabrics.

The functional composition herein can contain from about 0.001% to about 10%, alternatively from about 0.01 % to about 5%, alternatively from about 0.1% to about 2%, of cationic polymer, typically having a molecular weight of from about 500 to about 5,000,000 (although some cationic starches can be as high as 10,000,000 in molecular weight), alternatively from about 1,000 to about 2,000,000, alternatively from about 1,000 to about 1,000,000, and alternatively from about 2,000 to about 500,000 and a charge density of at least about 0.01 meq/gm., and up to about 23 meq/gm., alternatively from about 0.05 to about 8 meq/gm., alternatively from about 0.08 to about 7 meq/gm., and even alternatively from about 0.1 to about 1 milliequivalents/gram (meq/gm).

The cationic polymers of the present invention can be amine salts or quaternary ammonium salts. Preferred are quaternary ammonium salts. They include cationic derivatives of natural polymers such as some polysaccharide, gums, starch and certain cationic synthetic polymers such as polymers and copolymers of cationic vinyl pyridine or vinyl pyridinium halides. Preferably the polymers are water-soluble, for instance to the extent of at least 0.5% by weight are soluble in water at 20°C. Preferably the polymers have molecular weights (Daltons) of from about 500 to about 5,000,000, preferably from about 1,000 to about 2,000,000, more preferably from about 1,000 to about 1,000,000, and even more preferably from about 2,000 to about 500,000, and especially from about 2000 to about 100,000. As a general rule, the lower the molecular weight, the higher the degree of substitution (D.S.) by cationic, usually quaternary groups, which is desirable, or, correspondingly, the lower the degree of substitution, the higher the molecular weight which is desirable, but no precise relationship appears to exist. In general, the cationic polymers may have a charge density of at least about 0.01 meq/gm., preferably from about 0.05 to about 8 meq/gm., more preferably from about 0.08 to about 7 meq/gm., and even more preferably from about 0.1 to about 1 meq/gm. Cationic polymers are disclosed in U.S. Patent No. 6,492,322 at column 6, line 65 to column 24, line 24. Other cationic polymers are disclosed in the CTFA "International Cosmetic Ingredient Dictionary and Handbook," Tenth Edition, Tara E. Gottschalck and Gerald N. McEwen, Jr., editors, published by The Cosmetic, Toiletry, and Fragrance Association, 2004. Still other cationic polymers are described at U.S. Patent Publication 2003/0139312 A1, published July 24, 2003, from paragraph 317 to paragraph 347. Non-limiting examples of cationic polymers include those disclosed in WO 2008/120176.

In one embodiment, the cationic polymer comprises a polysaccharide gum. Of the polysaccharide gums, guar and locust bean gums, which are galactomannan gums are available commercially, and are preferred. In another embodiment, the cationic polymer comprises cationic guar gum. Guar gums are marketed under Trade Names CSAA M/200, CSA 200/50 by Meyhall and Stein-Hall, and hydroxyalkylated guar gums are available from the same suppliers. Other polysaccharide gums commercially available include: Xanthan Gum; Ghatti Gum; Tamarind Gum; Gum Arabic; and Agar. Cationic guar gums under the Trade Name N-Hance are available from Aqualon.

In one embodiment, the functional composition is free or substantially free of any cationic polymer.

e. Cationic Starches

In one embodiment, the functional material comprises a cationic polymer comprising a cationic polysaccharide. In another embodiment, the cationic polysaccharide comprises a cationic starch. The terms "polysaccharide" and "cationic starch" are used herein in the broadest sense. A cationic starch can also be used as a fabric care active, e.g., for softness and conditioning. Non-limiting examples of cationic starches are disclosed in U.S. Pat. Pub. 2004/0204337 and EP1989281.

**Adjunct Compositions**

**1. Viscosity/Hydrophobicity Modifiers**

In one embodiment the functionalized substrate further comprises modifiers including viscosity or hydrophobicity modifiers. In one embodiment, the modifiers are minimized. Typical viscosity modifiers include, but not limited to, silicone oil, gums, and waxes. Typical hydrophobic modifiers include, but not limited to, isopropyl myristate, mineral oil, dipropylenemethyl ether (DPM). Such modifiers may be used at less than 50%, alternatively less than 40%, alternatively less than 30%, alternatively less than 20%, alternatively less than 10%, alternatively less than 5%, alternatively less than 1%, alternatively about 0%, alternatively at least 0.1% but not greater than 50%, by weight of total perfume composition. Without wishing to be bound by theory, the overuse of modifiers reduces the efficiency of the scent experience imparted by the perfume microcapsules of the present invention.

**2. Solubility Modifiers**

In one embodiment, the functional substrate further comprises a solubility modifier. Solubility modifiers are substances which modify the solubility of the film and/or functional materials by for example delaying or accelerating its solubility or making solubility dependent of external factors such as pH, temperature, ionic strength, redox potential, surfactant concentration, etc. One example of a solubility modifier is an amino-acetylated polysaccharide, having a selected degree of acetylation. Other suitable solubility modifiers may include the polymers described in US 2003/0158072 A1, whose water solubility may be triggered by changes in pH, salt concentration, concentration of surfactant or a combination of both. The polymer is a copolymer or terpolymer containing from 2 to 60 mole % of a protonated amine functionality which has been neutralized with a fatty acid. WO 02/26928 provides non-limiting examples of suitable composite polymers that can be used for controlled release purposes, as in laundry.

Additional suitable solubility modifiers that are soluble in a given pH range are based on methacrylic acid co-polymers, styrene hydroxystyrene co-polymers, acrylate co-polymers, polyethylene glycol polyvinyl acetate, diethylphtalate, dioctyl sodium sulfocuccinate, poly-dl-lactide-co-glycolide (PLG), vinylpyridine/styrene co-polymers.

Solubility modifiers that are soluble in a specific chemistry environment are also commercially available. For instance caustic soluble barrier agents are commercially available from Alcoa under the trade name Hydra-Coat-5. Water dispersible barrier agents are based on sodium starch glycolate, polyplasdone and are commercially available from FMC Corporation under the trade name Ac-di-sol, from Edward Mendell Corporation under the trade name Explotab, from ISP under the trade name Crospovidone.

### 3. <u>Structurant</u>

In another embodiment, the functional substrate further comprises a structurant. Without intending to be bound by theory, it is believed that addition of a structurant helps the suspension of the microcapsules within the functionalized substrate. Acceptable for use herein are polymeric structurants selected from the group consisting of polyacrylates and derivatives thereof; polysaccharides and derivatives thereof; polymer gums and combinations thereof. Polyacrylate-type structurants comprise in particular polyacrylate polymers and copolymers of acrylate and methacrylate. An example of a suitable polyacrylate type structurant is Carbopol Aqua 30 available from B.F. Goodridge Company. Examples of polymeric gums which may be used as structurant herein can be characterized as marine plant, terrestrial plant, microbial polysaccharides and polysaccharide derivatives. Examples of marine plant gums include agar, alginates, carrageenan and furcellaran. Examples of terrestrial plant gums include guar gum, gum arable, gum tragacenth, karaya gum, locust bean gum and pectin. Examples of microbial polysaccharides include dextrin, gellan gum, rhamsan gum, welan gum and xanthan gum. Examples of polysaccharide derivatives include carboxymethyl cellulose, methyl hydroxypropyl cellulose, hydroxy propyl cellulose, hydroxyethyl cellulose, propylene glycol alginate and hydroxypropyl guar. The second structurant is selected from the above list or a combination thereof. Acceptable polymeric gums include pectin, alginate, arabinogalactan (gum Arabic), carrageenan, gellan gum, xanthan gum, Diutan® gum (ex. CP Kelco), and guar gum. If polymeric gum structurant is employed herein, an acceptable material of this type is gellan gum. Gellan gum is a tetrasaccharide repeat unit, containing glucose, glucurronic acid, glucose and rhamrose residues and is prepared by fermentation of Pseudomonaselodea ATCC 31461. Gellan gum is commercially marketed by CP Kelco U.S., Inc. under the KELCOGEL tradename.

In yet another embodiment, the functionalized substrate further comprises a plasticizer, for example glycerol, dipropylene glycol, ethylene glycol, diethyleneglycol, propylene glycol, sorbitol and mixtures thereof. Glycerol is one preferred plasticizer. Other useful additives include disintegrating aids.

**Functionalized Substrate Properties**

**1. Microcapsule Distribution**

In one embodiment, the plurality of microcapsules is incorporated with said functionalized substrate. In another embodiment, the plurality of microcapsules is dispersed throughout said functional substrate. As used herein, dispersed means that the microcapsules are present within the functional composition, including ordered and non-ordered dispersion patterns. In one embodiment, the plurality of microcapsules dispersed throughout said functional substrate is completely random. Further, the dispersed microcapsules are not printed, laminated or coated onto the functional substrate, rather the plurality of microcapsules are present within and throughout the functional substrate. In another embodiment, the plurality of microcapsules is uniformly dispersed throughout said functional substrate. In yet another embodiment, microcapsules are printed, laminated or coated onto the functional substrate that already contains a plurality of microcapsules dispersed throughout said functional substrate. The microcapsules which are printed, laminated or coated onto the functional substrate can be the same or different from the plurality of microcapsules dispersed throughout said functional substrate.

In one embodiment, the plurality of microcapsules is localized to a discreet area or areas of the functionalized substrate, such as in a pattern or design; alternatively in random discrete areas. In another embodiment, the plurality of microcapsules is not localized to any discreet area of the functionalized substrate.

**2. Volume Fraction**

In one embodiment of the present invention, the functionalized substrate comprises a volume fraction of microcapsules to functionalized substrate from about 0.1 to about 0.8, alternatively from about 0.2 to about 0.7, alternatively from about 0.3 to about 0.6, alternatively from about 0.4 to about 0.5, as calculated by the volume fraction calculation defined herein.

Volume Fraction Calculation Method:

Dissolve the article in water or an aqueous solution until no particulate is visually detected. Filter water solution using a preweighed Grade No. 44 Quantitative Filter Paper (particle retention of down to $3\mu m$, available from Whatman in Florham Park, New Jersey). Separate microcapsules from other insolubles in the filtrate by methods disclosed in the art. Let filter with microcapsules air dry to evaporate all the water and/or solvents. Weigh the dry filter paper to determine weight of microcapsules retained. Recover a small amount of the filtrated microcapsules and analyze using a particle sizer (examples are light microscopy and laser light scattering methods) and following manufacturer's instructions to obtain the mean particle diameter. Determine volume of average capsule ($V_{PMC}$). Assuming particle density equal to water, determine weight of an average capsule. Calculate the approximate number of capsules in filtrate ($N_{PMC}$). Use these values to calculate the volume fraction of capsules in the microcapsule containing article.

$$\phi_{PMC} = \frac{V_{PMC} \cdot N_{PMC}}{V_{article}}$$

Those of skill in the art will recognize that other methods of calculating volume fraction can be used without departing from the scope of the present invention.

### 3. Microcapsule Concentration

In one embodiment of the present invention, the functionalized substrate comprises a microcapsule concentration of from about $1 \times 10^6$ of microcapsules per 100 grams of said functionalized substrate to about $5 \times 10^{10}$ of microcapsules per 100 grams of said functionalized substrate, alternatively from about $1 \times 10^8$ of microcapsules per 100 grams of said functionalized substrate to about $1 \times 10^{10}$ of microcapsules per 100 grams of said functionalized substrate, alternatively from about $1 \times 10^9$ of microcapsules per 100 grams of said functionalized substrate to about $5 \times 10^9$ of microcapsules per 100 grams of said functionalized substrate.

### 4. Tensile Properties

In one embodiment of the present invention, the functionalized substrate comprises an elongation at break of from about 0 percent to about 800 percent, alternatively from about 1 percent to about 200 percent, alternatively from about 2 percent to about 50 percent. In another embodiment, the tensile stress at maximum load is from about 0 MPa to about 15 MPa, alternatively from about 1 MPa to about 5 MPa; alternatively from about 1.2 MPa to about 3MPa.

### 5. Dissolution Rate

As defined herein, dissolution rate means the rate at which the article dissolves when immersed in an aqueous solution. In one embodiment, the functionalized substrate comprises a rapid dissolution rate when said functionalized substrate is immersed in an aqueous solution at cold temperature, i.e. 4·4 or 10°C (40°F or 50°F) and standard atmospheric pressure. As used herein, rapid dissolution means that the functionalized substrate dissolves at a rate of from about 0.1 grams/minute to about 0.5 grams/minute, alternatively from about 0.15 grams/minute to about 0.4 grams/minute, alternatively from about 0.2 grams/minute to about 0.3 grams/minute. In another embodiment, the dissolution rate of the functionalized substrate is such that when the functionalized substrate is immersed into an aqueous solution during the wash and/or rinse cycles using a conventional washing machine, the functionalized substrate dissolves prior to the completion of the wash and/or rinse cycle.
Dissolution rate can be determined according to the Dissolution Rate Determination Method described herein in the Examples.

### 6. Basis Weight

In one embodiment, the functionalized substrate comprises a basis weight. Where the functionalized substrate comprises a film, the functionalized substrate has a basis weight from about 25 g/m$^2$ to about 150 g/m$^2$, alternatively from about 50 g/m$^2$ to about 100 g/m$^2$.

### Forms of the Functionalized Substrate

In one embodiment, the functionalized substrate is in any form which can be used to administer the article into the wash and/or rinse cycles in a conventional washing machine. Examples of suitable forms include: a film, a solid, a bar, a foam, an air-stable foam, or a laminated film.
In the laundry products embodiments of the present invention, the functionalized substrates can be used to separate functional materials from one another (in the case where certain functional materials are incompatible) as well as control the release or relative amounts of certain functional materials. The functionalized substrates can comprise more than one functional material in a layer or a plurality of layers comprising a plurality of functional materials or discrete regions comprising different functional materials.

## 1. Film Dimensions

In one embodiment of the present invention, the functional substrate comprises a film. In one embodiment, the film comprises a laminar or substantially planar form or sheet. As defined herein, substantially planar means that the film is in the form of a generally flat sheet which can be bent, folded, or otherwise deformed but still has the general shape of a flat sheet.

In one embodiment, the film comprising an average thickness of less than about 1 mm, alternatively less than about 0.5 mm, alternatively less than about 0.15 mm, alternatively less than about 0.1 mm, alternatively less than about 0.05 mm, alternatively less than about 0.04 mm, alternatively greater than about 0.01 mm. In one embodiment, the average thickness of the functionalized substrate comprises from about 0.025 mm to about 0.160 mm, alternatively from about 0.060 mm to about 0.130 mm.

In another embodiment, the functionalized substrate comprises wafers, foams, sponges, bars, noodle and pasta shapes, or other three dimensional forms. In these cases, the average thickness of the functionalized substrate can be greater; alternatively less than about 1 cm; alternatively less than about 0.5 cm.

Without intending to be bound by theory, it is believed that thicker substrates generally take more time to dissolve in aqueous solution than thinner ones. Although delayed release of perfumes is generally desired, the functionalized substrate cannot take so long dissolve as to stain laundry. In one embodiment, the functionalized substrate when placed in a load of laundry completely dissolves within the time frame of the laundry wash cycle (irrespective of water temperature). In another embodiment, the functionalized substrate when placed in a load of laundry completely dissolves within the time frame of the laundry rinse cycle (irrespective of water temperature).

The functionalized substrate is cut into or prepared in the form of small pieces, having a maximum linear dimension of from from 1 to 20 mm, as a stand alone product. The substrate may be cut or prepared into "confetti" that is added or incorporated as part of the powder, liquid or gel compositions. In another embodiment, the functionalized substrate is cut or formed into a shape which resembles, including but not limited to: flowers, flower petals, or leaves. Another embodiment can be in the form of a strip of film on a roll to dispense like tape with perforations to allow separation of segments of film. In one embodiment, the roll of film can be contained in a separate container or in a compartment of the closure; for example the closure of a bottle of liquid fabric conditioner and/or a liquid detergent product. Functionalized films are an effective way of protecting sensitive ingredients as well as controlling the delivery of functional materials. In order to provide additional protection, the cutting operation can performed such that no functional materials are potentially exposed on the edge of the cut pieces. This is particularly advantageous when the functionalized cut pieces are introduced in a product in liquid/gel form that can potentially react with the functional material exposed on the edge of the cut pieces.

In one embodiment, where the film is cut into pieces weighing from about 0.5 grams to about 5 grams, alternatively from about 1 gram to about 3 grams, alternatively from about 1.5 grams to about 2 grams.

Small pieces may be in the shape of squares, triangles, rectangles, circles, spherical beads, or other geometric shapes, or completely random, or combinations thereof. The small pieces each may have same color or a different color.

## 2. Substrate Layering

In one embodiment, the functionalized substrate comprises a single layer substrate. In one embodiment, the film comprises a multi-layer substrate.

In one embodiment where the film comprises multi-layers, the composition of a first layer of film is different than the composition of a second layer of film. In another embodiment, the functional material of a first layer of film is different than the functional material of a second layer of film. Examples of different functional materials can include embodiments where the first functional material comprises a PMC and the second functional material can be a functional material other than PMC; or where the first functional material comprises a first PMC and the second functional material comprises a second PMC, wherein the encapsulated perfume components are different. These different encapsulated perfumes components can have different chemical formulas or create different scent effects. In another embodiment, the present invention comprises more than two layers, wherein each layer comprises a functional material, and wherein at least two of the layers have different functional materials.

One advantage to having more than one layer is that a relatively less expensive film can be placed around a more expensive film, thereby lowering overall cost. Additionally, certain substrates may be capable of accommodating higher amounts of certain functional materials. By providing a multi-layers system, the combination of substrate layer to functional material can be optimized so that the layer is more accommodating to a specific functional material. Benefits for multi-layered systems include, but are not limited to, providing higher loading capacity, promoting substrate stability, isolating interaction between certain functional materials, etc. Further, by placing specific functional materials in specific layers the release of the functional materials into the wash and/or rinse can be controlled. For example, functional materials which are desirably released earlier in the wash and/or rinse can be placed on the outer layer(s), whereas functional materials which are desirably released later in the wash and/or rinse can be placed in the inner layer(s).

In another embodiment, the article comprises a first functionalized substrate and a second functionalized substrate. Where the article comprises more than one functional substrate, the compositions susceptible to aqueous attack, the microcapsules, and/or the functional compositions can be different across the different substrates in order to control compatibility as well as provide controlled release.

## 3. Foams

In one embodiment of the present invention, the functionalized substrate is in the form of a foam which is air-stable, but instable when contacted with water, i.e., dissolve in water. Suitable foam embodiments may be in a particle form of a sponge-like structure, used as a binder within the article or in sheet form to encapsulate or coat the article.

It has been found that when a specific foam component, comprising polymeric material and a functional composition according to the present invention is used, effective delivery of the active and protection of the active, not only against air-moisture and chemical reactions but also against physical forces, is achieved. The foam component is found to be air-stable under normal humidity storage conditions, but water-unstable (subject to aqueous attack) to thus deliver the actives, disintegrating or dissolving in water, to thus deliver the actives. Further, the foam may serve as a substrate for the active absorbing the active on its surface or adsorbing it into the cells of the foam. The functional actives can be in the form of a microcapsule or free active or both, and 2 or more actives can be used. In addition, the foam component can act as a binder providing structural integrity to the article. Further, the foam may be used as an outer coating to protect the article and prevent premature disintegration or dusting of the article.

The foam component is preferably a stable flexible foam. It is critical that the foam component be stable when in contact with air and yet unstable upon contact with water. The foam component preferably releases the active ingredient or part thereof upon contact with water, with the foam component preferably partially or completely disintegrating, dispersing, denaturing and/ or dissolving upon contact with water. The foam component may preferably be in the form of particles that can be incorporated in compositions, or in the form of a sheet, preferably such that it can form a foam sheet that can be used as protective coating for the composition.

Process for Making the Foam Component

The foam component may be made by any process known in the art for making foam components, preferably involving at least a step of mixing the composition susceptible to aqueous attack with the plurality of microcapsules. In one embodiment, the process comprises the steps of:

a) obtaining a composition susceptible to aqueous attack;

b) chemically or physically introducing gas in said composition;

c) prior to step b) and/or simultaneously with step b) and/ or subsequently to step b), addition of a plurality of microcapsules encapsulating a functional material;

d) optionally addition of further ingredients, preferably including a plasticizer and/or with an aqueous solution, in one or more of steps a), b) or c); and

e) optionally one or more of steps b), c) or d) followed by removal of the aqueous solution or part thereof.

Non-limiting examples of suitable foams of polymeric materials and methods of forming such foams and/or sponges have been disclosed in U.S. Patent Nos. 7,056,877, 4,824,582 and 6,033,729, which are incorporated herein.

## 4. Other Forms

In one embodiment of the present invention, the functionalized substrate further comprises a surfactant suitable for cosmetic use on skin or for cleaning of hard surfaces (such as dishes or floors) or skin. Suitable surfactants include soap, anionic surfactant, nonionic surfactant, amphoteric surfactant, zwitterionic, cationic surfactant and mixtures thereof. Further, in one embodiment, the functionalized substrate further comprises a carrier material. Suitable carrier materials include soluble or partially soluble starches, water soluble amorphous solids or semi-crystalline water soluble solids, and mixtures thereof. In one embodiment, the carrier material is a polyethylene glycol. In yet another embodiment, the article comprises a surfactant suitable for cosmetic use on skin and a functionalized substrate comprising a carrier material and a functional material comprising a microcapsule encapsulating a perfume. Non-limiting examples of said carrier material is a starch or a starch derivative, soap, and combinations thereof. In one embodiment article further comprises: propylene glycol, sorbitol, glycerin, sodium laureth sulfate, sodium stearate, sodium myristate, sodium cocoyl isethionate, triethanolamine, water, and a perfume microcapsule.

**Coordinating Substrate Shape and Benefit**

In one embodiment of the present invention, the functionalized substrate, in any of the above disclosed forms, comprises a shape, wherein the shape and the benefit and/or at least one functional material are coordinated. By shape, it is also meant that the form of the functionalized substrate can include any drawings, wording and/or colorations which can be placed on the functionalized substrate by any method known in the art. This includes scent and shape names as well as trademarks. As used herein, coordinated means any relationship between the shape and the consumer desired benefit and/or functional material, such that a consumer would understand that the shape represents the benefit (scent or other benefits) and/or the functional material or vice versa. For example, one type of coordination would be where the functionalized substrate was shaped as an orange where the benefit is a citrus scent or where the substrate is shaped as a flower where the benefit is floral scent. In one embodiment where the plurality of microcapsules encapsulates a perfume and where the perfume provides a specific scent experience, the shape of the functionalized substrate is coordinated with the scent experience generated by the perfume.

Non-limiting examples of coordinated shapes and scent experiences include any natural or artificial combinations of shape and scent, generally flower blossoms or flower petals. Other naturally occurring combinations of shape and scent include, but are not limited to: rose, rose petal, lavender, lilac, sunflower, canola flower, daisy, tulip, daffodil, dahlia, honeysuckle, honey comb, morning glory, jasmine, water lily, lily, carnation, orchid, white orchid, lotus flower, magnolia, violet, pansy, orange, lemon, lime, apricot, tangerine, plum, mandarin, mango, kiwi, apple, peach, pear, cherry, grape, cucumber, lavender, vanilla, vanilla bean, coffee bean, aloe vera plant, chamomile, eucalyptus, peony, gardenia, white gardenia, ylang, lily of valley, hyacinth, linden blossom, osmanthus, tuberose, orange flower, persimmon, bergamot, banana, strawberry, blueberry, gooseberry, raspberry, blackberry, juniper berry, rhubarb, papaya, guava, passion fruit, grapefruit, white grapefruit, pink grapefruit, boysenberry, watermelon, honeydew melon, cantaloupe, pomegranate, tea leaves, white tea, pine/pinecone, cedar, maple leaf, oak, ash, elm, rain drop, slices of any of the above mentioned fruits or vegetables), palm tree, cherry blossom, clover, cinnamon stick, ivy, water drop, ocean wave, white lilac, lemon verbena, rice flower, ginger flower, cotton blossom, milk & honey, linen flower, oats, and sweet pea. Artificial combinations of shape and scent include, but are not limited to: talcum powder scent with baby bundle, bottle, stuffed animals, linens, pillows, and bedding, bath and towel shapes; new car small with automobile shapes, morning dew scent with rain drops, dew drops, sun, moon, star, or cloud shapes.

Process of making Co-casted substrates

According to another aspect of the present invention, there is provided a process of making functionalized substrates according to the present invention. The process comprises the steps of:

> a. combining said composition which is susceptible to aqueous attack with said plurality of microcapsules to form a combination;
> b. mixing said combination at low shear mixing to form a mixture;
> c. deaerating said mixture to form an at least partially deaerated mixture;
> d. casting said at least partially deaerated mixture to form a casted at least partially deaerated mixture; and
> e. drying said casted at least partially deaerated mixture to produce a functionalized substrate.

As defined herein, an at least partially deaerated mixture means that if there are any air bubbles present in the mixture at least some of the air entrapped within the mixture to escape, thereby decreasing the occurrence of air bubble formation within the mixture. In one embodiment, the functionalized substrate is free of any air bubbles having an air bubble diameter from about 1 cm to about 1 micron, alternatively from about 1 mm to about 5 microns, alternatively from about 0.5 mm to about 25 microns, alternatively from about 0.1 mm to about 50 microns, alternatively from about 0.05 mm to about 200 microns, alternatively from about 0.01 mm to about 300 microns. In another embodiment, said low shear mixing comprises any degree of mixing which causes a minimal amount of the plurality of microcapsules to rupture, for example mixing the mixture in a beaker with a magnetic stir bar at a low speed, such as below 300 rpm. In another embodiment, where making functionalized substrates in the form of foams or sponges, the step of deaerating may be skipped. Further, in one embodiment, the composition which is susceptible to aqueous attack is available by from the steps of

a) obtaining a water-soluble film;

b) dissolving said water soluble film in an aqueous solution; and

c) evaporating at least a portion of said aqueous solution to reach the desired composition.

**Examples:**

**1. Making a Functionalized Substrate comprising PVA**

Apparatus:

An apparatus which can be used to make functional substrates according to the present invention is the Gardco Automatic Drawdown Machine DP-8201 (available from Paul N. Gardner Company, Inc. in Pompano Beach, Florida. The Gardco Automatic Drawdown Machine casting table is equipped with casting bars of different depths and glass slides.

Obtaining a composition susceptible to aqueous attack:

- Obtain a volume of polyvinyl alcohol (PVA) resin. Alternatively, M8630 polyvinyl alcohol 1.5ml thickness film (available from MonoSol LLC in Merrillville, Indiana) can be converted into a PVA solution by addition of the film and water into a metal beaker. Mix manually with spatula until most of the film is dissolved. Place beaker into a water bath, heat to 70°C and mix using an overhead mixer until the film is fully dissolved in the water. Water can be added in excess to help dissolve the film faster. Once the film is dissolved, allow the water to evaporate to the desired weight. Allow the mixture to cool to room temperature.

- Add the PVA resin solution or PVA film solution to a microcapsule slurry

- Mix at low speed (less than 300 rpm) for 10 minutes.

- Let the formula deaerate overnight to avoid the formation of air bubbles in the film.

Film Casting:

- Place a transparency sheet (glossy side up) over a glass slide to serve as a carrier sheet for the casted film.

- Put 5 layers of masking tape in the shape of a box around the borders of the transparency sheet (with dimensions of 24.5 cm x 20.0 cm). The number tape layers will determine the thickness of the film.

- Treat the surface of the transparency with isopropyl alcohol to remove oily residues.

- Cover the surface of the transparency with a thin layer of non-ionic surfactant (e.g. Poly-Tergent SLF18 available from BASF Corporation in Mount Olive, New Jersey) to aid with the release of the film.

- Place the glass slide over the casting table, secure it using the clamp and dispense 60 to 65 grams of the slurry at the inner edge of the right side of the tape box.

- Turn on the drawdown machine by switching the power button to on.

- Secure the ends of the wire wrapped 58/90 casting bar into the slots provided in the bar holder and roll it over so it lays flat over the right side of the tape box next to the slurry.

- Set the drawdown machine settings to:

○ Speed Adjust - 11.5 setting

○ Stroke Length - 12 inches

- Press the Start Test button to slide the casting bar through the glass slide and spread the slurry. If the slurry was not spread evenly, press the Manual Reset button to spread the slurry backwards.

- Remove the glass slide from the clamp and let the casted film air dry for 4 to 6 hours.

- Peel the tape off the glass slide and carefully release the casted film from the carrier sheet. If the film does not peel easily, use a razor blade to cut smoothly against the transparency to peel the film.

| Ingredient | Function | I | II | III |
|---|---|---|---|---|
| | | % | % | % |
| M8630 PVOH 1.5mil Film | Water Soluble Film Carrier | 15.29 | 19.17 | 20.85 |
| Glycerol | PVOH Film Plasticizer *(from film)* | 1.68 | 2.11 | 2.29 |
| Appleton PMCs | PMC Mixture *(composition as follows)* | 73.03 | 67.71 | 65.65 |
| Perfume oil | Perfume | *[53.49]* | *[49.60]* | *[48.09]* |
| Urea/formaldehyde | Capsule Wall | *[9.43]* | *[8.74]* | *[8.47]* |
| Ethyl Acetoacetate | Formaldehyde Scavenger | *[10.04]* | *[9.30]* | *[9.02]* |
| Acticide MBS[a] | Preservative | *[0.04]* | *[0.04]* | *[0.04]* |
| Sanolin Violet E2R | Product Color | *[0.04]* | *[0.04]* | *[0.04]* |
| Mirapol 550[b] | Deposition Aid | --- | 1.01 | --- |
| Guar Gum[c] | Deposition Aid | --- | --- | 1.21 |
| DI Water | | 10.00 | 10.00 | 10.00 |
| *TOTAL* | | 100.00 | 100.00 | 100.00 |

[a]Microbiocide based on isothiazolinones available from Acti-Chem Specialties Inc. in Trumbull, Connecticut.
[b]Aqueous cationic copolymer with a typical molecular weight between 900,000 and 1,000,000 available from Rhodia Novecare in Cranbury, New Jersey.
[c]Terrestrial plant gum mainly consisting of high molecular weight (50,000-8,000,000) polysaccharides composed of galactomannans available from Sigma-Aldrich in St. Louis, Missouri.

## 2. Making a Functionalized Substrate comprising Starch

The process for making a starch based functional substrate is similar to the process for making a PVA based functional substrate. One example includes: adding water and starch into a glass beaker and cooking at 90°C for an hour while stirring with a magnetic stir bar. Adding the plasticizer and M8630 PVA film (or PVA resin) into the hot starch solution and mix. Allow the solution to cool to room temperature. Adding PMC and all other ingredients and mix at low speed (less than about 300 rpm) for about 10 minutes. Letting the formula deaerate for up to 12 hours to avoid the formation of air bubbles in the film.

| Ingredient | Function | IV | V | VI |
|---|---|---|---|---|
| | | % | % | % |
| Cargill Superfilm 270W[d] | Water Soluble Film Carrier | 34.53 | 16.76 | 19.80 |
| Glycerol | Plasticizer | 34.53 | 15.85 | 6.60 |

(continued)

| Ingredient | Function | IV | V | VI |
|---|---|---|---|---|
| | | % | % | % |
| M8630 PVOH 1.5mil Film | Water Soluble Film Carrier | 3.10 | 1.99 | 2.12 |
| Glycerol | PVOH Film Plasticizer *(from film)* | 0.34 | 0.22 | 0.23 |
| Appleton PMCs | PMC Mixture *(composition as follows)* | 17.50 | 55.18 | 61.25 |
| Perfume oil | Perfume | *[12.80]* | *[40.42]* | *[44.86]* |
| Urea/formaldehyde | Capsule Wall | *[2.26]* | *[7.12]* | *[7.91]* |
| Ethyl Acetoacetate | Formaldehyde Scavenger | *[2.40]* | *[7.58]* | *[8.42]* |
| Acticide MBS | Preservative | *[0.01]* | *[0.03]* | *[0.03]* |
| Sanolin Violet E2R | Product Color | *[0.03]* | *[0.03]* | *[0.03]* |
| DI Water | | 10.00 | 10.00 | 10.00 |
| **TOTAL** | | 100.00 | 100.00 | 100.00 |
| [d]Oxidized corn starch available from Cargill Inc. in Minneapolis, Minnesota. | | | | |

### 3. Film Dissolution Testing

Dissolution Rate Determination Method:

Apparatus:

- 600mL beaker of 100mm diameter

- Magnetic stirrer

- Magnetic stirrer rod (5cm)

- Thermometer (0 to 100°C) with precision of 1 °C

- Timer, accurate to nearest second

- Polaroid 35 mm x 23 mm slide frame (external size: 50 mm x 50 mm)

- Slide mount holder

- Distilled water

Reagents & Solutions: Industrial/city water

Procedure:

The procedure to assess the disintegration time of water-soluble films was adapted from MonoSol Test Method 205 (ASTM 205).

Cut three test specimens from a functionalized substrate sample. Measure the thickness of each film sample using a micrometer. Place each specimen in a separate 35mm slide frame and avoid wrinkles in the film. The orientation of the film in the frame has no impact on the measurement.

Fill the 600mL beaker with 500mL of city/industrial water. Measure the water temperature with thermometer and, if necessary, heat or cool to maintain temperature at 40°F (about 4°C). Control the temperature to ± 1°C. Record the temperature of the water.

Place the beaker on magnetic stirrer, add magnetic stirring rod to beaker, turn on stirrer, and adjust the stir speed to control the vortex height so the final water level is four fifths of the original water height (when the bottom of the vortex is at the 400ml mark). Mark depth of vortex.

Secure the 35mm slide frame in the alligator clamp of the slide mount holder such that the long end of the slide frame is parallel to the water surface. The slide should be placed so that the film surface is perpendicular to the flow of the water.

In one motion, drop the secured slide and clamp into the water and start the timer. The slide must be 5 mm $\pm$ about 1 mm away from the wall. The short side of the frame should be parallel with the side of the beaker. The film should be totally immersed in the water.

The time for a film to first break is called the *disintegration time* (or rupture time). *Dissolution* occurs when virtually all film fragments are no longer visible. The time for the film to release from the slide mount into the water as free particles is called the *dispersion time.* Do not remove the slide from the water until the test is finished since this will influence the dissolution time. Record disintegration and dissolution times for three replicates from each film sample.

Dissolution Test Results at 4.44°C (40°F)

|                         | I   | II    | III | V   | VI  |
| ----------------------- | --- | ----- | --- | --- | --- |
| Film thickness ($\mu$m) | 211 | 185   | 173 | 340 | 234 |
| Disintegration Time (min.) | 0.8 | 0.009 | 0.7 | 1.6 | 0.8 |
| Dissolution Time (min.) | 2.2 | 2.2   | 4.5 | --- | --- |
| Dispersion Time (min.)  | --- | ---   | --- | 3.1 | 5.0 |

### 4. Petal Forming

Films from Examples I - VI are cut into a petal shape approximately 1-2 grams in weight with a length of about 100 mm and a width a the widest point of about 70 mm with a die cutter.

### 5. Article Performance Testing

Articles cut into the form of petals from Example I were added through the wash cycle under standard North America washing conditions and using a machine dryer:

<u>Apparatus:</u>

○ Washer: Kenmore 80 Series (Model 26-26952)
○ Dryer: Kenmore (Model 26-66922)

Washer Settings:

○ 64.35 L (17 gallon) water fill
○ Single rinse
○ 12 minute cycle
○ Agitation set to "Normal" (slow, fast)
○ City water at 32.2°C (90°F) for the wash cycle
○ City water at 15.56°C (60°F) for the rinse cycle

Dryer Settings:

○ Cotton High Heat setting
○ 50 minutes timer

99.4g of Tide Free® Liquid Detergent
5.5 pounds of stripped fabric ballast

Three different petal quantities (1, 3, and 5 petals) were compared to a control treatment of no petals and evaluated for wet and dry fabric odor performance on 100% cotton terries. The results from this evaluation showed that the addition of a petal to the wash cycle provides a significant increase in wet and dry fabric odor. Increasing the number of petals dosed into the wash provides an incremental benefit on fabric odor. Additionally, the Petal treated terries continued to provide a significant dry fabric odor benefit for up to two weeks, providing increased scent longevity.

The same petal doses were evaluated under identical washing conditions by adding the petals during the rinse cycle instead of the wash cycle of the laundry process. The fabric odor evaluation for the petals added in the rinse cycle showed a higher increase in wet and dry fabric odor than observed for the petals dosed during the wash cycle.

**Methods of Using Articles**

The present invention also provides for a laundry bath solution prepared by dispensing in a generally aqueous laundry bath, one or more articles according to the present invention. The resultant solution may comprise a pre-soak or wash or rinse cycle solutions prepared in an automated washing machine, manual washing device, tub or other container. The solution may optionally contain a detergent and/or fabric softening composition.

After the fabric care composition has been dispensed in the laundry solution, it is preferred that the laundry solution contain between about 0.1ppm and about 500 ppm of the functional composition.

The functional compositions, articles and dosing are as described in detail throughout this disclosure. A unitized dose or article may be dispensed directly into a wash and/or rinse bath solution. Articles according to the present invention are applicable in aqueous solution across a broad range of pH levels, in both warm and cold water solutions, and when other materials are present in the solution.

Where the article is to be dispensed into a rinse bath solution, but dispensing is desired at the beginning of the wash cycle, the article or dose may be placed in dispensing means for delayed dispensing. Dispensing means will include the dispensing devices that are built into commercially available washing machines such as dispensing drawers and top loaded agitator dispensers. Likewise, the dispensing means will also include self-contained dispensing devices that may be placed in the tub of the machine at the start of the wash cycle (one example is the Downy® Ball). As previously described, the self-contained dispensing devices that are particularly useful in the methods of the present invention are those that are designed to open during the spin cycle that follows the wash and precedes the rinse cycle. When a self-contained dispensing device is used to dispense an article or dose, it is preferred that water or a liquid fabric softening composition also be added to the dispenser to aid in the dissolution and dispensing of the fabric care composition. More specifically, it is preferred that between about 5ml and about 150ml of water and/or liquid fabric softener be added to the self-contained device.

The method of preparing a customized laundry solution will optionally include the use of a scented or unscented detergent and/or fabric softener composition. Because it is anticipated that consumers will want the opportunity to choose the fragrance that will be deposited on their fabrics, it is preferred that such detergent and fabric softening compositions be unscented. The optional detergent and/or fabric softening composition may be any detergent or fabric softener that is known in the art and may be unitized or a measured amount of a bulk composition.

The present invention allows users to design their own customized scent experience by blending two or more functionalized substrates where the substrates can contain different perfumes, with or without the additional usage of conventional laundry products. For example, the user can select a first functionalized substrate with a citrus scent (with or without a corresponding shape) and a second functionalized substrate with a floral scent (with or without a corresponding shape). Additionally, the users can use one or more functionalized substrates in combination to select mixtures of desired benefits, such as using one or more functionalized substrates selected to provide a specific customized scent experience with one or more functionalized substrates selected to provide a non-scent benefit, such as softness, anti-static, anti-wrinkle, fiber cleaning, etc.

The method of preparing a customized laundry solution will optionally include the step of providing information to the consumer which may assist the consumer in selecting a fabric care composition, or an article or dose containing such a composition, that will deliver a desired fabric care benefit. This information is preferably provided in the form of instructions that may be used to guide the consumer as has been described herein in conjunction with the articles and laundry kits of the present invention.

Articles of the present invention may be designed for dosing into an automatic dish washing machine. Such articles can provide scent in the washing process (i.e., fragrance into the kitchen) and/or other functional benefits (such as cleaning from surfactants, bleaches, enzymes, solvents, soli dispersing polymers, chelants, and the like). Other functional benefits can be rinsing aids (to avoid water spotting) and antifoams (so higher levels of cleaning surfactants can be used). The benefit active agents can be in microcapsules, as well as free, or combinations thereof. Preferably the functionalize substrate is added at the start of the washing cycle and dissolves and/or disperses in the pre-rinse cycle. Alternatively the functionalized substrate dissolves and/or disperses in the wash cycle or in the final rinse cycle.

It should be understood that every maximum numerical limitation given throughout this specification includes every lower numerical limitation, as if such lower numerical limitations were expressly written herein. Every minimum numerical limitation given throughout this specification includes every higher numerical limitation, as if such higher numerical limitations were expressly written herein. Every numerical range given throughout this specification includes every narrower numerical range that falls within such broader numerical range, as if such narrower numerical ranges were all expressly written herein.

All parts, ratios, and percentages herein, in the Specification, Examples, and Claims, are by weight and all numerical limits are used with the normal degree of accuracy afforded by the art, unless otherwise specified.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

All documents cited in the DETAILED DESCRIPTION OF THE INVENTION are, in the relevant part, incorporated herein by reference; the citation of any document is not to be construed as an admission that it is prior art with respect to the present invention. To the extent that any meaning or definition of a term or in this written document conflicts with any meaning or definition in a document incorporated by reference, the meaning or definition assigned to the term in this written document shall govern.

While particular embodiments of the present invention have been illustrated and described, it would be obvious to those skilled in the art that various other changes and modifications can be made without departing from the scope of the invention. It is therefore intended to cover in the appended claims all such changes and modifications that are within the scope of this invention.

**Claims**

1. An article comprising:

   a. a functionalized substrate comprising:

      i. a composition susceptible to aqueous attack, wherein said composition is selected from water-soluble materials, partially water soluble materials, water-dispersible materials, water-disintegrating materials, and mixtures thereof; and
      ii. a plurality of microcapsules encapsulating a functional composition,

   wherein said plurality of microcapsules is incorporated with said functionalized substrate,

   wherein the functionalized substrate is cut or prepared in the form of small pieces having a maximum linear dimension of from 1 to 20 mm, as a stand alone product,
   wherein the functional composition comprises a perfume composition,
   and wherein said plurality of microcapsules comprises a friable microcapsule.

2. The article according to Claim 1 or 2 wherein said functional composition comprises a second functional material comprising selected from flavors, softening agents, anti-static agents, crisping agents, water/stain repellents, stain release agents, refreshing agents, anti-microbial agents, disinfecting agents, wrinkle resistant agents, wrinkle release agents, odor resistance agents, malodor control agents, abrasion resistance and protection agents, solvents, insect/pet repellents, wetting agents, UV protection agents, skin/fabric conditioning agents, skin/fabric nurturing agents, color protection agents, dye fixatives, dye transfer inhibiting agents, silicones, preservatives and anti-microbials, fabric shrinkage-reducing agents, perfume microcapsules, brighteners, hueing dyes, bleaches, chelants, antifoams, anti-scum agents, whitening agents, and combinations thereof.

3. The article according to any of the preceding claims, wherein said composition comprises polyvinyl alcohol, derivatives of polyvinyl alcohol, carboxymethyl cellulose, derivatives of carboxymethyl cellulose, hydroxypropyl methylcellulose, polyoxyethylene, starch, starch derivative, surfactant, soap, polyethylene glycol, and mixtures thereof.

4. The article according to any of the preceding claims, wherein the functionalized substrate is formed into a shape, wherein said shape and said perfume are coordinated.

5. A process of making a functionalized substrate according to any of the preceding claims, comprising the steps of:

a. combining said composition which is susceptible to aqueous attack with said plurality of microcapsules to form a combination;
b. mixing said combination at low shear mixing;
c. deaerating said mixture to form an at least partially deaerated mixture;
d. casting said at least partially deaerated mixture to form a casted at least partially deaerated mixture; and
e. drying said casted at least partially deaerated mixture to produce a functionalized substrate.

**Patentansprüche**

1. Gegenstand, der Folgendes umfasst:

a. ein funktionalisiertes Substrat, das Folgendes umfasst:

I. eine Zusammensetzung, die von Wasser angegriffen wird, wobei die Zusammensetzung ausgewählt ist aus wasserlöslichen Materialien, teilweise wasserlöslichen Materialien, wasserdispergierbaren Materialien, wasserzersetzbaren Materialien und Mischungen davon, und
II. mehrere Mikrokapseln, die eine funktionelle Zusammensetzung einschließen,

wobei die mehreren Mikrokapseln in das funktionalisierte Substrat eingegliedert sind,
wobei das funktionalisierte Substrat in Form kleiner Stücke einer linearen Höchstabmessung von 1 bis 20 mm, als eigenständiges Produkt, geschnitten bzw. hergestellt wird,
wobei die funktionelle Zusammensetzung eine Duftstoffzusammensetzung umfasst
und wobei die mehreren Mikrokapseln eine krümelige Mikrokapsel umfassen.

2. Gegenstand nach Anspruch 1 oder 2, wobei die funktionelle Zusammensetzung ein zweites funktionelles Material ausgewählt aus Geschmacksstoffen, Weichmachern, antistatischen Mitteln, Verfestigungsmitteln, Wasser-/Fleckenabweisern, Fleckenlösemitteln, Auffrischern, antimikrobiellen Mitteln, Desinfektionsmitteln, Knitterschutzmitteln, Entknitterungsmitteln, Geruchsresistenzmitteln, Geruchsunterdrückungsmitteln, Abriebbeständigkeits- und Abriebschutzmitteln, Lösemitteln, Insekten-/Haustierabwehrmitteln, Benetzungsmitteln, UV-Schutzmitteln, Haut-/Stoffpflegemitteln, Haut-/Stoffnährmitteln, Farbschutzmitteln, Farbfixierern, Farbstoffübertragungshemmern, Silikonen, Konservierungsmitteln und antimikrobiellen Mitteln, Gewebeschrumpfhemmern, Duftstoff-Mikrokapseln, Aufhellern, Abtönungsfarbstoffen, Bleichmitteln, Chelatbildnern, Schaumverhinderern, Schmutzschaumverhinderern, Weißmachern und Kombinationen davon umfasst.

3. Gegenstand nach einem der vorstehenden Ansprüche, wobei die Zusammensetzung Polyvinylalkohol, Derivative von Polyvinylalkohol, Carboxymethylcellulose, Derivative von Carboxymethylcellulose, Hydroxypropylmethylcellulose, Polyoxyethylen, Stärke, Stärkederivat, Tensid, Seife, Polyethylenglykol und Mischungen davon umfasst.

4. Gegenstand nach einem der vorstehenden Ansprüche, wobei das funktionalisierte Substrat in eine Form gebracht wird, wobei die Form und der Duftstoff aufeinander abgestimmt werden.

5. Verfahren zur Herstellung eines funktionalisierten Substrats nach einem der vorstehenden Ansprüche, das folgende Schritte umfasst:

a. das Vermischen der Zusammensetzung, die durch Wasser angegriffen wird, mit den mehreren Mikrokapseln zum Bilden einer Verbindung,
b. das Vermischen der Verbindung unter Mischung bei niedriger Scherkraft,
c. das Entlüften der Mischung zum Bilden einer zumindest teilweise entlüfteten Mischung,
d. das Gießen der zumindest teilweise entlüfteten Mischung, um eine zumindest teilweise entlüftete gegossene Mischung zu bilden, und
e. das Trocknen der gegossenen, zumindest teilweise entlüfteten Mischung zur Herstellung eines funktionalisierten Substrats.

**Revendications**

1. Article comprenant :

   a. un substrat fonctionnalisé comprenant :

   i. une composition prédisposée à une attaque aqueuse, où ladite composition est choisie parmi des matériaux hydrosolubles, des matériaux partiellement hydrosolubles, des matériaux hydrodispersibles, de matériaux se désintégrant dans l'eau, et leurs mélanges ; et
   ii. une pluralité de microgélules encapsulant une composition fonctionnelle,

   dans lequel ladite pluralité de microgélules est incorporée avec ledit substrat fonctionnalisé,
   dans lequel le substrat fonctionnalisé est coupé ou préparé sous la forme de petites pièces ayant une dimension linéaire maximale allant de 1 à 20 mm, en tant que produit autonome,
   dans lequel la composition fonctionnelle comprend une composition parfumée, et dans lequel ladite pluralité de microgélules comprend une microgélule friable.

2. Article selon la revendication 1 ou 2, dans lequel ladite composition fonctionnelle comprend un deuxième matériau fonctionnel choisi parmi les arômes, agents adoucissants, agents antistatiques, agents amidonnants, repellants de l'eau/des taches, agent de libération des salissures, agents de rafraîchissement, agents antimicrobiens, agents désinfectants, agents de résistance aux plis, agents de libération des plis, agents de résistance aux odeurs, agents de contrôle de mauvaise odeur, agents de résistance à l'abrasion et de protection, solvants, repellants d'insectes/ animaux de compagnie, agents mouillants, agents de protection UV, agent de conditionnement de la peau/des tissus, agent d'entretien de la peau/des tissus, agents de protection des couleurs, fixateurs de colorants, agents inhibant la décoloration, silicones, conservateurs et antimicrobiens, agents réduisant le rétrécissement des tissus, microgélules de parfum, azurants, teintures teintantes, agents de blanchiment, agents chélatants, antimousses, agents anti-écume, agents procurant de la blancheur, et leurs combinaisons.

3. Article selon l'une quelconque des revendications précédentes, dans lequel ladite composition comprend un alcool polyvinylique, des dérivés d'alcool polyvinylique, de la carboxyméthylcellulose, des dérivés de carboxyméthylcellulose, de l'hydroxypropyl-méthylcellulose, la polyoxyéthylène, l'amidon, un dérivé d'amidon, un agent tensioactif, un savon, le polyéthylène glycol, et leurs mélanges.

4. Article selon l'une quelconque des revendications précédentes, dans lequel le substrat fonctionnalisé est coupé ou formé en une forme, ladite forme et ledit parfum étant coordonnés.

5. Procédé de fabrication d'un substrat fonctionnalisé selon l'une quelconque des revendications précédentes, comprenant les étapes consistant à :

   a. combiner ladite composition qui est prédisposée à une attaque aqueuse à ladite pluralité de microgélules de façon à former une combinaison ;
   b. mélanger ladite combinaison par un mélange par faible cisaillement ;
   c. désaérer ledit mélange de façon à former un mélange au moins partiellement dégazé ;
   d. mouler ledit mélange au moins partiellement dégazé de façon à former un mélange au moins partiellement dégazé moulé ; et
   e. sécher ledit mélange au moins partiellement dégazé moulé pour produire un substrat fonctionnalisé.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 3936538 A, Marshall
- US 7049274 B, Renade
- US 7015186 B, Aussant
- US 20050226826 A, Eason
- US 20060019866 A, Briggs
- US 7056877 B
- US 20060293419 A1
- US 7094817 B
- WO 0131103 A3
- US 6211309 B
- US 5224601 A
- JP 3525174 B
- JP H09279457 B
- JP H10008364 B
- US 2003215417 A1
- US 2003216488 A1
- US 2003158344 A1
- US 2003165692 A1
- US 2004071742 A1
- US 2004071746 A1
- US 2004072719 A1
- US 2004072720 A1
- EP 1393706 A1
- US 2003203829 A1
- US 2003195133 A1
- US 2004087477 A1
- US 20040106536 A1
- US 6645479 B
- US 6200949 B
- US 4882220 A
- US 4917920 A
- US 4514461 A
- US RE32713 E
- US 4234627 A
- US 5246603 A
- US 3516941 A
- US 4520142 A
- US 4528226 A
- US 4681806 A
- US 4145184 A
- GB 2073132 A
- WO 9917871 A
- US 20040087477 A1
- EP 2021172 A, Wahl
- US 20050192207 A1
- US 5500138 A
- US 20020035053 A1
- EP 1141207 A
- US 5082578 A
- US 20050020476 A1
- US 20060217288 A1
- US 20060058214 A1
- WO 2008120176 A
- US 6492322 B
- US 20030139312 A1
- US 20040204337 A
- EP 1989281 A
- US 20030158072 A1
- WO 0226928 A
- US 4824582 A
- US 6033729 A

### Non-patent literature cited in the description

- MICROENCAPSULATION: Methods and Industrial Applications. Marcel Dekker, Inc, 1996
- **S. ARCTANDER.** Perfume and Flavor Chemicals (Aroma Chemicals. 1969
- **A. J. GORDON ; R. A. FORD.** The Chemist's Companion. John Wiley & Sons Publishers, 1972, 30-36
- **D. T. STANTON et al.** Computer-Assisted Prediction of Normal Boiling Points of Pyrans and Pyrroles. *J. Chem. Inf. Comput. Sci.,* 1992, vol. 32, 306-316
- **D. T. STANTON et al.** Computer-Assisted Prediction of Normal Boiling Points of Furans,'' Tetrahydrofurans, and Thiophenes. *J. Chem. Inf. Comput. Sci.,* 1992, vol. 31, 301-310
- **R. MURUGAN et al.** Predicting Physical Properties from Molecular Structure. *Chemtech,* June 1994, 17-23
- **MCCUTCHEON'S.** Functional Materials, North American. the Manufacturing Confectioner Publishing Company, 1997, vol. 2
- International Cosmetic Ingredient Dictionary and Handbook. The Cosmetic, Toiletry, and Fragrance Association, 2004